# EUROPEAN PATENT APPLICATION

(11) **EP 3 747 996 A1**
(43) Date of publication of application: **09.12.2020**
(21) Application number: 19748057.7
(22) Date of filing: 31.01.2019
(51) Int. Cl.: C12N 5/10, C07K 14/50, C12N 9/99

(54) **METHOD FOR PRODUCING CELLS**

(30) Priority: 31.01.2018 JP 2018015008; 30.01.2019 JP 2019014484
(71) Applicant: FUJIFILM Corporation, Tokyo 106-8620 (JP)
(72) Inventor: YAMAZAKI, Nao, Kanagawa 258-8577 (JP); MURAKAMI, Yuta, Kanagawa 258-8577 (JP); HOSOYA, Masaki, Kanagawa 258-8577 (JP); MURAGUCHI, Taichi, Kanagawa 258-8577 (JP)
(74) Representative: Hoffmann Eitle
(86) International application number: PCT/JP2019/003336
(87) International publication number: WO 2019/151386

(57) **Abstract**

An object of the present invention is to provide a method for producing a pluripotent stem cell that can efficiently differentiate into a target differentiated cell. According to the present invention, a method for producing a cell is provided, the method including a first step of obtaining an undifferentiated cell which is obtained by introducing a reprogramming factor into a somatic cell and has a relatively low ability to differentiate into a specific differentiated cell; and a second step of treating the cell while maintaining an undifferentiated state of the cell to obtain a cell having a relatively high ability to differentiate into the specific differentiated cell.

## Description

### Field of the Invention

The present invention relates to a method for producing a cell having an ability to differentiate into any one or more of an endoderm, a mesoderm, or an ectoderm.

### Description of the Related Art

As pluripotent stem cells, induced pluripotent stem cells (also referred to as iPS cells), embryonic stem cells (also referred to as ES cells), and the like are known. In the field of regenerative medicine, research for practical use of iPS cells has been particularly advanced.

It is known that pluripotent stem cells such as iPS cells include naive cells and primed cells. Naive cells are closer to the early developmental state, whereas primed cells are developmentally more advanced cells.

Non-Patent Document 1 discloses a method of leading pluripotent stem cells to revert by a histone deacetylase inhibitor to convert the cells into naïve cells.

Patent Document 1 discloses a method for inducing non-pluripotent mammalian cells to become induced pluripotent stem cells, the method including a stage of bringing non-pluripotent cells into contact with a TGF-β receptor/ALK5 inhibitor; a MEK inhibitor; and a ROCK inhibitor under conditions sufficient to induce cells to become pluripotent stem cells; and discloses that the cells are further brought into contact with a histone deacetylase inhibitor. Patent Document 2 discloses a method for inducing non-pluripotent mammalian cells to become induced pluripotent stem cells, the method including a step of bringing non-pluripotent cells into contact with a 3'-phosphoinositide-dependent-kinase-1 (PDK1) activator under conditions sufficient to induce cells to become pluripotent stem cells, whereby inducing non-pluripotent mammalian cells into induced pluripotent stem cells; and discloses that the method may further include a step of bringing the non-pluripotent cells into contact with a histone deacetylase inhibitor.

Patent Document 3 discloses a method for producing naive pluripotent stem cells, the method including: temporarily expressing Nanog and Klf2 in primed pluripotent stem cells; and culturing the cells in a medium containing a LIF, a MEK inhibitor, a GSK3 inhibitor, a cAMP production promoter, a TGF-β inhibitor, and a PKC inhibitor.

Patent Document 4 discloses a method of leading human stem cells to revert to a more naive state, the method including: (a) providing human stem cells to be reprogrammed; (b) (i) expressing or introducing one or more optionally different types of reprogramming factors into the cells, and (ii) culturing the cells in a medium for reverting which contains a MEK inhibitor, optionally a STAT3 activator, and optionally one or more another inhibitors to induce a more naive state; and (c) retaining the cells in a naive medium containing a MEK inhibitor, a PKC inhibitor and optionally a GSK3 inhibitor, and a STAT3 activator.

### Prior Art Documents

### Patent Documents

Patent Document 1: JP2016-027808A
Patent Document 2: JP2016-171798A
Patent Document 3: WO2016/148253A
Patent Document 4: WO2016/027099A

### Non-Patent Documents

Non-Patent Document 1: Guo G. et al. Development, 2017 Aug 1; 144 (15): 2748-2763

### SUMMARY OF THE INVENTION

Based on the previous studies, the inventors of the present invention have found that there are iPS cells that cannot differentiate into all of three germ layers among iPS cells produced by introducing a reprogramming factor into somatic cells even in a case where a marker is expressed as an iPS cell.

An object of the present invention is to provide a method for producing a pluripotent stem cell that can efficiently differentiate into a target differentiated cell.

The inventors of the present invention have conducted intensive studies to achieve the above-mentioned object, and as a result, they have succeeded in producing pluripotent stem cells that can efficiently differentiate into target differentiated cells by obtaining an undifferentiated cell which is obtained by introducing a reprogramming factor into a somatic cell and has a relatively low ability to differentiate into a specific differentiated cell, and then treating the cell while maintaining an undifferentiated state of the cell to obtain a cell having a relatively high ability to differentiate into the specific differentiated cell. The present invention has been completed based on the above findings.

That is, according to the present invention, the following inventions are provided.
(1) A method for producing a cell, comprising:
   a first step of obtaining an undifferentiated cell which is obtained by introducing a reprogramming factor into a somatic cell and has a relatively low ability to differentiate into a specific differentiated cell; and
   a second step of treating the cell while maintaining an undifferentiated state of the cell to obtain a cell having a relatively high ability to differentiate into the specific differentiated cell.
(2) The method according to (1), in which the ability of the cell obtained in the second step to differentiate into any one or more of an endoderm, a mesoderm, or an ectoderm is more improved than the ability of the cell obtained in the first step to differentiate into any one or more of an endoderm, a mesoderm, or an ectoderm.
(3) The method according to (1),
   in which the ability of the cell obtained in the second step to differentiate into each of an endoderm and an ectoderm is more improved than the ability of the cell obtained in the first step to differentiate into each of an endoderm and an ectoderm, or
   the ability of the cell obtained in the second step to differentiate into each of an endoderm and a mesoderm is more improved than the ability of the cell obtained in the first step to differentiate into each of an endoderm and a mesoderm.
(4) The method according to (2) or (3), in which the cell obtained in the second step is a cell capable of differentiating into all of an endoderm, a mesoderm, and an ectoderm.
(5) The method according to (1), in which the specific differentiated cell is a mesodermal cell or an ectodermal cell.
(6) The method according to (1), in which the specific differentiated cell is a hemocyte, a cardiomyocyte, or a neurocyte.
(7) The method according to any one of (1) to (6), in which an expression level of STELLA in the cell obtained in the second step is higher than an expression level of STELLA in the cell obtained in the first step.
(8) The method according to any one of (1) to (7), in which an expression level of KLF17 in the cell obtained in the second step is higher than an expression level of KLF17 in the cell obtained in the first step.
(9) The method according to any one of (1) to (8), in which the second step is a step of treating the cell with a histone deacetylase inhibitor while maintaining the undifferentiated state of the cell.
(10) The method according to (9), in which the second step is a step of treating the cell with the histone deacetylase inhibitor and a basic fibroblast growth factor while maintaining the undifferentiated state of the cell.
(11) The method according to (9) or (10), in which the second step includes a step of culturing the cell in a medium containing the histone deacetylase inhibitor, a MAPK/ERK kinase inhibitor, and a leukemia inhibitory factor, and then culturing the cell in a medium not containing the histone deacetylase inhibitor but containing the MAPK/ERK kinase inhibitor, a protein kinase C inhibitor, a Wnt signaling inhibitor, and the leukemia inhibitory factor.
(12) The method according to any one of (9) to (11), in which the histone deacetylase inhibitor is valproic acid or a salt thereof.
(13) The method according to any one of (1) to (12), in which a medium in the second step does not contain ascorbic acid.
(14) The method according to any one of (1) to (13), in which the somatic cell is a human adult somatic cell.
(15) The method according to any one of (1) to (14), further comprising a third step of differentiating the cell obtained in the second step.
(16) A method for producing a pluripotent stem cell having an improved ability to differentiate into three germ layers as compared to a primed pluripotent stem cell, the method comprising a step of treating the primed pluripotent stem cell with a histone deacetylase inhibitor while maintaining an undifferentiated state of the cell.
(17) A method for producing a pluripotent stem cell having an improved ability to differentiate into a specific differentiated cell as compared to a primed pluripotent stem cell, the method comprising a step of treating the primed pluripotent stem cell with a histone deacetylase inhibitor while maintaining an undifferentiated state of the cell.
(18) The method according to (17), in which the specific differentiated cell is a hemocyte, a cardiomyocyte, or a neurocyte.
(19) The method according to any one of (16) to (18), in which the step of treating the primed pluripotent stem cell with the histone deacetylase inhibitor while maintaining the undifferentiated state of the cell includes a step of culturing the cell in a medium containing the histone deacetylase inhibitor, a MAPK/ERK kinase inhibitor, and a leukemia inhibitory factor, and then culturing the cell in a medium not containing the histone deacetylase inhibitor but containing the MAPK/ERK kinase inhibitor, a protein kinase C inhibitor, a Wnt signaling inhibitor, and the leukemia inhibitory factor.
(20) The method according to any one of (16) to (19), in which the histone deacetylase inhibitor is valproic acid or a salt thereof.
(21) The method according to any one of (16) to (20), in which a medium in the step of treating the primed pluripotent stem cell with the histone deacetylase inhibitor while maintaining the undifferentiated state of the cell does not contain ascorbic acid.

According to the present invention, it is possible to produce a pluripotent stem cell that can efficiently differentiate into a target differentiated cell.

### BRIEF DESCRIPTION OF THE DRAWINGS

Fig. 1 shows results of quantitative RT-PCR for measuring expression of genes defining an undifferentiation state in untreated and treated human iPS cell lines (the line 253G1).
Fig. 2 shows results of quantitative RT-PCR for measuring expression of genes specific to each of germ layers in cells (the line 253G1) differentiated into three germ layer lineages.
Fig. 3 shows results of quantitative RT-PCR for measuring expression of genes specific to each of germ layers in another iPS cell lines (the line 201B7 and the line A) differentiated into three germ layer lineages.
Fig. 4 shows results obtained by respectively differentiating untreated and treated iPS cells into hemocytes, and analyzing a proportion of cells expressing CD34 and KDR, which are markers of hemocytes, by a flow cytometer.
Fig. 5 shows results of quantitative RT-PCR for evaluating an ability of iPS cells to differentiate into three germ layers.
Fig. 6 shows a summary of the evaluation results of the ability of the iPS cells to differentiate into three germ layers.
Fig. 7 shows results of flow cytometry for evaluating treatment efficiency in a case where a concentration of a MEK inhibitor was changed.
Fig. 8 shows results of evaluating the number of copies of a specific region of a chromosome in a case where a concentration of the MEK inhibitor was changed.
Fig. 9 shows results obtained by respectively inducing untreated and treated iPS cells into cardiomyocytes, and comparing cTnT-positive percentages in living cells by flow cytometry.
Fig. 10 shows results of measuring cTnT-positive percentages in a case where an iPS cell line was induced into cardiomyocytes.
Fig. 11 shows images obtained by observing cells obtained by being differentiation-induced into neural stem cells with a fluorescence microscope.
Fig. 12 shows results of quantifying brightness of the images obtained by observing the cells obtained by being differentiation-induced into neural stem cells with the fluorescence microscope.

### DESCRIPTION OF THE PREFERRED EMBODIMENTS

Hereinafter, embodiments for carrying out the present invention will be described in detail.

Abbreviations in the present specification have the following meanings.
Klf: Kruppel-like factor
LIF: Leukemia inhibitory factor
MEK: MAPK/ERK kinase (MAPK: mitogen-activated protein kinase; ERK: extracellular signal-regulated kinase)
GSK3: glycogen syn-thase kinase-3
TGF: Transforming growth factor
PKC: Protein kinase C
RT-PCR: Reverse transcription polymerase chain reaction
PCR: Polymerase chain reaction
KDR: Kinase insert domain-containing receptor
Tert: Telomerase reverse transcriptase
Fbx15: F-Box protein 15
ECAT: ES cell associated transcripts
Dnmt3L: DNA methyltransferase 3 like
Gdf3: Growth differentiation factor-3
Fthl17: Ferritin heavy polypeptide-like 17
Sall4: Sal-like protein 4
Rex1: Reduced-expression 1
UTF1: Undifferentiated embryonic cell transcription factor 1
Stat3: Signal transducer and activator of transcription 3
Grb2: Growth factor receptor-bound protein 2
Prdm14: PR/SET domain family 14
Nr5a1: Nuclear receptor subfamily 5, group A, member 1
Nr5a2: Nuclear receptor subfamily 5, group A, member 2
bFGF: Basic fibroblast growth factor
POU5F1: POU domain, class 5, transcription factor 1
DNMT3B: DNA (cytosine-5-)-methyltransferase 3 beta
GAPDH: Glyceraldehyde 3-phosphate dehydrogenase
FOXA2: Forkhead box protein A2
T: Brachyury
PDGFRA: Platelet-derived growth factor receptor alpha
PAX6: Paired box 6
MAP: Mitogen-activated protein
BMP: Bone morphogenetic protein
VEGF: Vascular endothelial growth factor
IL: Interleukin
IGF: Insulin-like growth factor
SCF: Stem cell factor
ROCK: Rho-associated coiled-coil forming kinase/Rho-binding kinase
ALK5: TGF-beta type I receptor
CD34: Cluster of differentiation 34
Sox: SRY-boxes
ERas: ES cell expressed Ras
Tell: T-cell leukemia/lymphoma 1A

As pluripotent stem cells, cells in two different states are known: naive pluripotent stem cells and primed pluripotent stem cells. Naive pluripotent stem cells and primed pluripotent stem cells can be distinguished by molecular and cellular characteristics.

Naive pluripotent stem cells typically have characteristics of expressing high levels of pluripotent factors Oct4, Nanog, Sox2, Klf2, and Klf4; self-renewing in response to any one of Lif/Stat3 or 2i (ERKi/GSKi); differentiating in response to Fgf/Erk; and exhibiting XaXa for the active status of X chromosomes. Primed pluripotent stem cells typically have characteristics of expressing high levels of pluripotent factors Oct4, Sox2, and Nanog; self-renewing in response to Fgf/Erk without responding to Lif/Stat3; and exhibiting XaXi for the active status of X chromosomes (Nichols et al., (2009) Cell Stem Cell 4 (6): 487-492). Xa indicates an active X chromosome, and Xi indicates an inactive X chromosome.

A method for producing a cell according to a first embodiment of the present invention includes:
a first step of obtaining an undifferentiated cell which is obtained by introducing a reprogramming factor into a somatic cell and has a relatively low ability to differentiate into a specific differentiated cell; and
a second step of treating the cell while maintaining an undifferentiated state of the cell to obtain a cell having a relatively high ability to differentiate into the specific differentiated cell. Cells subjected to the same operation under the same culture conditions are regarded as cells having the same properties even though they are different individuals.

A method for producing a cell according to a second embodiment of the present invention is a method for producing a pluripotent stem cell having an improved ability to differentiate into three germ layers or an improved ability to differentiate into a specific differentiated cell as compared to a primed pluripotent stem cell, the method including a step of treating the primed pluripotent stem cell with a histone deacetylase inhibitor while maintaining an undifferentiated state of the cell.

JP2016-027808A and JP2016-171798A disclose that non-pluripotent cells are brought into contact with a histone deacetylase inhibitor in a case where the cells are induced to become pluripotent cells. WO2016/148253A discloses that naïve pluripotent stem cells are produced by culturing primed pluripotent stem cells under predetermined conditions. However, there is no perception that there are iPS cells that cannot differentiate into all of three germ layers among iPS cells produced by introducing a reprogramming factor into somatic cells even in a case where a marker is expressed as an iPS cell. WO2016/027099A discloses the method of leading human stem cells to revert to a more naive state and discloses that the obtained stem cells differentiate into neurocytes, an endoderm, and smooth muscle cells, but it does not disclose that reverting to a naive state improves an ability to differentiate. In addition, the medium in WO2016/027099A is a medium containing ascorbic acid.

The present invention have identified that there are cells having a relatively low ability to differentiate into specific differentiated cells among undifferentiated cells obtained by introducing a reprogramming factor into somatic cells. In addition, the present invention have succeeded in treating the cells while maintaining an undifferentiated state thereof to obtain cells having a relatively high ability to differentiate into the specific differentiated cells.

### [Regarding first step]

The first step in the present invention is a step of obtaining an undifferentiated cell which is obtained by introducing a reprogramming factor into a somatic cell and has a relatively low ability to differentiate into a specific differentiated cell.

The somatic cell is not particularly limited, and any somatic cell can be used. For example, human adult somatic cells (that is, mature somatic cells) may be used in addition to fetal somatic cells. Examples of somatic cells include (1) tissue stem cells (somatic stem cells) such as neural stem cells, hematopoietic stem cells, mesenchymal stem cells, and dental pulp stem cells; (2) tissue precursor cells; and (3) differentiated cells such as fibroblasts (skin cells and the like), epithelial cells, hepatocytes, lymphocytes (T cells, B cells), endothelial cells, muscle cells, hair cells, gastric mucosal cells, intestinal cells, splenocytes, pancreatic cells (exocrine pancreas cells and the like), brain cells, lung cells, kidney cells, and skin cells.

A living body from which somatic cells are derived is not particularly limited, and example thereof include humans, and non-human animals (such as monkeys, sheep, cows, horses, dogs, cats, rabbits, rats, and mice). Humans are preferable.

The reprogramming factor to be introduced into somatic cells is not particularly limited. Examples thereof include Oct3/4, Klf4, c-Myc, Sox2, Nanog, Klf2, L-Myc, N-Myc, Klf5, Lin28, Tert, Fbx15, ERas, ECAT15-1, ECAT15-2, Tell, β-catenin, ECAT1, Esg1, Dnmt3L, ECAT8, Gdf3, Sox15, Fthl17, Sall4, Rex1, UTF1, Stella, Stat3, Grb2, Prdm14, Nr5a1, Nr5a2, and E-cadherin. Two or more genes can be selected from this group of genes and introduced in any combination. Among combinations, a combination having at least Oct3/4, Sox2, Klf4, and c-Myc; a combination having at least Oct3/4, Sox2, Klf4, and L-Myc; or a combination having at least Oct3/4, Sox2, Nanog, and Lin28 is preferable.

In addition, a species of a gene to be introduced is preferably the same as a species of a cell into which the gene is to be introduced. For example, a gene to be introduced into a human-derived cell is preferably a human gene. For example, as a gene to be introduced into a human-derived somatic cell, a combination having at least human Oct3/4, human Sox2, human Klf4, and human c-Myc; a combination having at least Oct3/4, Sox2, Klf4, and L-Myc; or a combination having at least human Oct3/4, human Sox2, human Nanog, and human Lin28 is preferable.

A gene as a reprogramming factor can be introduced into a somatic cell using a gene expression vector. The gene expression vector is not particularly limited, and examples thereof include a viral vector, a plasmid vector, an artificial chromosome vector, and a transposon vector. Examples of viral vectors include a retroviral vector, an adenovirus vector, a Sendai virus vector, a lentiviral vector, and an adeno-associated virus vector.

An undifferentiated cell which is obtained by introducing a reprogramming factor into a somatic cell and has a relatively low ability to differentiate into a specific differentiated cell may be prepared by introducing a reprogramming factor into a somatic cell. Alternatively, cells provided or sold by research institutions or companies may be purchased. That is, the first step in the present invention may be a step of obtaining an induced pluripotent stem cell from a bank for induced pluripotent stem cells.

For example, it is possible to purchase and use 201B7, 253G1, 253G4, 1201C1, 1205D1, 1210B2, 1231A3, 1383D2, 1383D6, iPS-TIG120-3f7, iPS-TIG120-4f1, iPS-TIG114-4f1, CiRA086Ai-m1, CiRA188Ai-M1, or iRA188Ai-W1, all of which are provided by Center for iPS Cell Research and Application, Kyoto University.

In addition, it is possible to purchase cells from iPS cell banks provided by National Institutes of Health (NIH), California Institute for Regenerative Medicine, New York Stem Cell Foundation, European Bank for induced pluripotent Stem Cells, and the like.

An undifferentiated cell referred to in the "undifferentiated cell which is obtained by introducing a reprogramming factor into a somatic cell" refers to a cell that has not yet fully differentiated and preferably refers to a cell that has an ability to differentiate into any one or more of an endoderm, a mesoderm, or an ectoderm. A specific differentiated cell referred to in the "cell having a relatively low ability to differentiate into a specific differentiated cell" refers to any one of an endoderm, a mesoderm, or an ectoderm, or refers to any specific differentiated cell derived from any one of an endoderm, a mesoderm, or an ectoderm. Examples thereof include hemocytes, cardiomyocytes, or neurocytes. The phase "a relatively low ability to differentiate into a specific differentiated cell" means that an ability of a cell to differentiate into a specific differentiated cell is low as compared to an ability of a "cell which has a relatively high ability to differentiate into a specific differentiated cell" and which is to be obtained in the second step of the present invention to be described later.

The "undifferentiated cell which is obtained by introducing a reprogramming factor into a somatic cell and has a relatively low ability to differentiate into a specific differentiated cell" and which is to be obtained in the first step can be maintained and cultured in an appropriate medium on a plate coated with feeder cells or a plate coated with a scaffold such as Matrigel (registered trademark). The feeder cells are not particularly limited, and examples thereof include mouse embryonic fibroblasts (MEF cells) and mouse SIM embryonic fibroblasts (STO cells).

As the medium for the maintenance culture, it is possible to use commercially available media such as mTeSR (registered trademark) 1 (STEMCELL Technologies Inc.) or StemFlex (registered trademark). Alternatively, examples of media include media prepared by supplementing any of basal media with any combination of additional components such as substitute serum (Knockout™ Serum Replacement (KSR) (Invitrogen)), fetal bovine serum (FBS), non-essential amino acids (NEAA), L-glutamine, 2-mercaptoethanol, antibiotics (such as streptomycin, penicillin, puromycin, and mitomycin), and bFGF, where examples of the above-mentioned basal media include Dulbecco Modified Eagle medium (DMEM), a mixed medium of DMEM and F12 (DMEM/F12 = 1:1), Knockout™ D-MEM (Invitrogen), and the like.

A medium for the maintenance culture preferably does not contain ascorbic acid.

Culture conditions for the maintenance culture are preferably conditions of 37°C, 5% CO₂, and 10% O₂, and the like, but they are not particularly limited.

### [Regarding second step]

The second step in the present invention is a step of treating the cell obtained in the first step while maintaining an undifferentiated state thereof to obtain a cell having a relatively high ability to differentiate into a specific differentiated cell. Hereinafter, in a case where the second step is referred to, the step includes a "step of treating a cell with a histone deacetylase inhibitor while maintaining an undifferentiated state thereof' in a second embodiment of the present invention.

A specific differentiated cell referred to in the "cell having a relatively high ability to differentiate into a specific differentiated cell" refers to any specific differentiated cell belonging to any one of an endoderm, a mesoderm, or an ectoderm. The phase "a relatively high ability to differentiate into a specific differentiated cell" means that an ability of a cell to differentiate into a specific differentiated cell is high as compared to an ability of the "cell which has a relatively low ability to differentiate into a specific differentiated cell" and which is obtained in the first step of the present invention.

The phase "treating while maintaining an undifferentiated state" means that the cell obtained in the first step is cultured in a medium, in which an undifferentiated state of the cell can be maintained, under conditions in which a cell having a relatively high ability to differentiate into a specific differentiated cell can be obtained.

The second step is preferably a step of treating the cell obtained in the first step with a histone deacetylase inhibitor while maintaining the undifferentiated state of the cell. That is, the second step is preferably a step including culturing the cell obtained in the first step in a medium containing the histone deacetylase inhibitor.

The second step is more preferably a step of treating the cell obtained in the first step with the histone deacetylase inhibitor and a basic fibroblast growth factor while maintaining the undifferentiated state of the cell.

The medium in the second step (or the step of treating the cell with the histone deacetylase inhibitor while maintaining the undifferentiated state of the cell) preferably does not contain ascorbic acid.

Examples of histone deacetylase inhibitors include valproic acid or salts thereof (such as sodium valproate), butyric acid or salts thereof (such as sodium butyrate), trichostatin A, apicidin, and the like, but examples are not particularly limited thereto.

A concentration of the histone deacetylase inhibitor in the medium can be appropriately set according to the type of histone deacetylase inhibitor, and the like. For example, in the case of valproic acid, a concentration is preferably 0.1 mmol/L to 10 mmol/L, more preferably 0.2 mmol/L to 5 mmol/L, and even more preferably 0.5 mmol/L to 2 mmol/L.

Examples of basal media, which are used for preparing the medium used in the step of treating the cell obtained in the first step with the histone deacetylase inhibitor while maintaining the undifferentiated state of the cell, include Dulbecco Modified Eagle medium (DMEM), a mixed medium of DMEM and F12 (DMEM/F12 = 1:1), Knockout™ D-MEM (Invitrogen), and the like. A medium used in the second step is preferably a medium obtained by supplementing the above-mentioned basal medium with any combination of additional components (preferably all of the additional components), and further adding a LIF and a MEK inhibitor, where examples of the additional components include Neurobasal (registered trademark) (Thermo Fisher Scientific), B27 (registered trademark) (Thermo Fisher Scientific), N2 (Thermo Fisher Scientific), 1-thioglycerol, GlutaMAX (registered trademark) (Thermo Fisher Scientific) or L-Glutamine (Thermo Fisher Scientific), and the like.

Examples of MEK inhibitors include PD0325901 (N-[(2R)-2,3-dihydroxypropoxy]-3,4-difluoro-2-[(2-fluoro-4-iodophenyl)amino]-benzamide; CAS Registry Number: 391210-10-9), U0126 (1,4-diamino-2,3-dicyano-1,4-bis[2-aminophenylthio]butadiene; CAS Registry Number: 109511-58-2), PD98059 (2-(2-amino-3-methoxyphenyl)-4H-1-benzopyran-4-one; CAS Registry Number: 167869-21-8), and PD184352 (2-(2-chloro-4-iodophenylamino)-N-cyclopropylmethoxy-3,4-difluorobenzamide; CAS Registry Number: 212631-79-3), but examples are not particularly limited thereto. Among the examples, PD0325901 is preferable.

The second step may include culturing the cell obtained in the first step in a medium not containing a histone deacetylase inhibitor after culturing the cell in a medium containing a histone deacetylase inhibitor. As the medium not containing a histone deacetylase inhibitor, it is possible to use a medium obtained by supplementing the above-mentioned basal medium with any combination of additional components (preferably all of the additional components), and further adding a LIF, a MEK inhibitor (specific examples thereof are as described above), a PKC inhibitor, a GSK3-β inhibitor, and a Wnt signaling inhibitor, where examples of the additional components include Neurobasal (registered trademark) (Thermo Fisher Scientific), B27 (registered trademark) (Thermo Fisher Scientific), N2 (Thermo Fisher Scientific), 1-thioglycerol, GlutaMAX (registered trademark) (Thermo Fisher Scientific) or L-Glutamine (Thermo Fisher Scientific), and the like.

The second step (or the step of treating the cell with the histone deacetylase inhibitor while maintaining the undifferentiated state of the cell) preferably includes a step of culturing the cell in a medium containing the histone deacetylase inhibitor, a MAPK/ERK kinase inhibitor, and a leukemia inhibitory factor, and then culturing the cell in a medium not containing the histone deacetylase inhibitor but containing the MAPK/ERK kinase inhibitor, a protein kinase C inhibitor, a Wnt signaling inhibitor, and the leukemia inhibitory factor.

Examples of PKC inhibitors include Go6983 (3-[1-[3-(dimethylamino)propyl]-5-methoxy-1H-indol-3-yl]-4-(1H-indol-3-yl)-1H-pyrrole-2,5 -dione; CAS Registry Number: 133053-19-7), and GF109203X (3-(1-(3-dimethylamino)propyl)-1H-indol-3-yl)-4-(1H-indol-3-yl)-1H-pyrrole-2,5-dione; CAS Registry Number: 133052-90-1), but examples are not particularly limited thereto. Among the examples, Go6983 is preferable.

The GSK3-β inhibitor is not particularly limited, but it is preferably CHIR99021 (CAS Registry Number: 252927-06-9).

Examples of Wnt signaling inhibitors include XAV939 (a tankyrase inhibitor) (CAS Registry Number: 284028-89-3); IWP-1, IWP-2, IWP-3, IWP-4, IWR-1, and 53AH (which are all porcupine inhibitors); low molecular compounds such as KY02111 and derivatives thereof; and proteins such as IGFBP4, DKK1, and Wnt-C59, but examples are not particularly limited thereto. Among the examples, XAV939 is preferable.

A concentration of a LIF in a medium is not particularly limited, but it is, for example, 0.1 ng/mL to 100 ng/mL, and preferably 0.2 ng/mL to 10 ng/mL.

A concentration of a MEK inhibitor in a medium is not particularly limited, but it is, for example, 50 nmol/L to 100 µmol/L, preferably 100 nmol/L to 10 µmol/L, and more preferably 200 nmol/L to 5 µmol/L, even more preferably 500 nmol/L to 2 µmol/L, and particularly preferably 800 nmol/L to 1.2 µmol/L.

A concentration of a PKC inhibitor in a medium is not particularly limited, but it is, for example, 50 nmol/L to 100 µmol/L, and preferably 100 nmol/L to 10 µmol/L.

A concentration of a GSK3-β inhibitor in a medium is not particularly limited, but it is preferably 0 nmol/L to 0.3 nmol/L.

A concentration of a Wnt signaling inhibitor in a medium is not particularly limited, but it is, for example, 50 nmol/L to 100 µmol/L, and preferably 100 nmol/L to 10 µmol/L.

Culture conditions in the second step are obvious to those skilled in the art, and examples thereof include conditions of 37°C and 5% CO₂. It is preferable that culturing be performed under low oxygen conditions (5% O₂).

A culture period in the second step is not particularly limited, and for example, the culture can be performed for 1 day to 14 days, and preferably 2 days to 14 days.

A CD75 (ST6GAL1), which is a specific cell surface marker, is generally known as an indicator for efficiency of reversion to a naive state. Accordingly, completion of the treatment in the second step can be evaluated by a positive percentage of the cell surface marker CD75. A positive percentage of the CD75 can be analyzed by flow cytometry by recovering cells treated in the second step from a culture vessel, and staining the cells with a fluorescently labeled anti-CD75 antibody (for example, an Alexa Fluor 647 Mouse Anti-Human CD75 antibody). A positive percentage of the CD75 may be, for example, 20% or more, 30% or more, 40% or more, 50% or more, 60% or more, or 70% or more.

In addition, a cell obtained after the treatment in the second step preferably has a low incidence of DNA mutations. A low incidence of DNA mutations can be evaluated by, for example, measuring the number of copies of a long arm region of chromosome 20 by a conventional method (for example, real-time PCR or the like). The number of copies of a long arm region of chromosome 20 in the cell obtained after the treatment in the second step is preferably 1.5 to 4.5, more preferably 1.5 to 3.5, and even more preferably 1.5 to 2.5.

An undifferentiation state of the cell obtained in the first step and the cell obtained in the second step is not particularly limited, and it can be evaluated by measuring expression of genes defining an undifferentiation state. A method of measuring expression of genes defining an undifferentiation state is not particularly limited, and measurement can be performed by, for example, quantitative RT-PCR. RT-PCR is a method of synthesizing cDNA using mRNA that is a measurement target as a template, and amplifying the cDNA by PCR using this cDNA as a template. Examples of quantitative RT-PCR include a method (real-time PCR) in which a PCR is performed using a primer to which a fluorescent quencher dye and a fluorescent reporter dye are bonded to quantify an amount of amplified product in each cycle, and measuring an amount of template DNA in a sample from the number of cycles in which detected fluorescence intensity sharply increases; and the like. Quantitative RT-PCR techniques are well known in the present technical field, and quantitative RT-PCR can also be performed using commercially available kits. According to quantitative RT-PCR, an expression level or the number of copies of a gene can be measured as a relative value with respect to an expression level or the number of copies of a control housekeeping gene (for example, GAPDH gene). The measurement of mRNA of a gene can be performed by subjecting amplified products obtained by amplifying mRNA by common RT-PCR or the like to gel electrophoresis, staining, and then measuring band intensity. Alternatively, mRNA or cDNA of a gene can be detected or quantified using DNA chip.

Genes defining an undifferentiation state are not particularly limited, and examples thereof include NANOG, POU5F1, LIN28, SOX2, DNMT3B, STELLA, KLF17, and the like.

An expression level of STELLA in the cell obtained in the second step is preferably higher than an expression level of STELLA in the cell obtained in the first step.

An expression level of KLF17 in the cell obtained in the second step is preferably higher than an expression level of KLF17 in the cell obtained in the first step.

An expression level of SOX2 in the cell obtained in the second step is preferably higher than an expression level of SOX2 in the cell obtained in the first step.

The number of copies of STELLA expressed in the cell obtained in the second step is preferably 0.001 or more, more preferably 0.002 or more, even more preferably 0.003 or more, and particularly preferably 0.004 or more, as a ratio to the number of copies of GAPDH expressed.

The number of copies of KLF17 expressed in the cell obtained in the second step is preferably 5.0 × 10⁻⁵ or more, more preferably 1.0 × 10⁻⁴ or more, even more preferably 1.1 × 10⁻⁴ or more, and particularly preferably 1.2 × 10⁻⁴ or more, as a ratio to the number of copies of GAPDH expressed.

The number of copies of SOX2 expressed in the cell obtained in the second step is preferably 5.0 × 10⁻⁵ or more, more preferably 6.0 × 10⁻⁵ or more, even more preferably 7.0 × 10⁻⁵ or more, and particularly preferably 8.0 × 10⁻⁵ or more, as a ratio to the number of copies of GAPDH expressed.

An ability of the cell obtained in the second step to differentiate into any one or more of an endoderm, a mesoderm, or an ectoderm is preferably more improved than an ability of the cell obtained in the first step to differentiate into any one or more of an endoderm, a mesoderm, or an ectoderm.

The ability of the cell obtained in the second step to differentiate into each of an endoderm and an ectoderm is more preferably more improved than the ability of the cell obtained in the first step to differentiate into each of an endoderm and an ectoderm, or the ability of the cell obtained in the second step to differentiate into each of an endoderm and a mesoderm is more preferably more improved than the ability of the cell obtained in the first step to differentiate into each of an endoderm and a mesoderm.

The cell obtained in the second step is even more preferably a cell capable of differentiating into all of an endoderm, a mesoderm, and an ectoderm.

The specific differentiated cells in the present invention are preferably mesodermal cells or ectodermal cells, and more preferably hemocytes, cardiomyocytes, or neurocytes.

In addition, regarding the improvement in ability to differentiate into three germ layers in the present invention, it is sufficient as long as an ability to differentiate into any one or more of an endoderm, a mesoderm, or an ectoderm is improved, but it is preferable that an ability to differentiate into any one or more of a mesoderm or an ectoderm be improved, and it is more preferable that an ability to differentiate into a mesoderm and an ectoderm be improved.

In the present invention, an ability of a cell to differentiate into an endoderm, an ability of a cell to differentiate into a mesoderm, and an ability of a cell to differentiate into an ectoderm can be evaluated by differentiating a cell into an endoderm, a mesoderm, or an ectoderm, and measuring expression of genes specific to each of the germ layers in the cell differentiated into the above three germ layer lineages.

A method of measuring expression of genes specific to each of the germ layers is not particularly limited, and measurement can be performed by, for example, a quantitative RT-PCR method.

Examples of endoderm-specific genes include SOX17, FOXA2, and the like, but examples are not particularly limited thereto.

Examples of mesoderm-specific genes include T, PDGFRA, and the like, but examples are not particularly limited thereto.

Examples of ectoderm-specific genes include PAX6, MAP2, and the like, but examples are not particularly limited thereto.

Examples of cases, in which the ability of the cell obtained in the second step to differentiate into an endoderm is more improved than the ability of the cell obtained in the first step to differentiate into an endoderm, include cases in which a relative expression level of SOX17 in a cell obtained by differentiating the cell obtained in the second step into an endoderm is preferably 2 or more, and more preferably 4 or more, 6 or more, 8 or more, 10 or more, 12 or more, or 15 or more with respect to an expression level of SOX17 in a cell obtained by differentiating the cell obtained in the first step into an endoderm.

Examples of cases, in which the ability of the cell obtained in the second step to differentiate into an endoderm is more improved than the ability of the cell obtained in the first step to differentiate into an endoderm, include cases in which a relative expression level of FOXA2 in a cell obtained by differentiating the cell obtained in the second step into an endoderm is preferably 3 or more, and more preferably 5 or more, 10 or more, 15 or more, 18 or more, 20 or more, or 22 or more with respect to an expression level of FOXA2 in a cell obtained by differentiating the cell obtained in the first step into an endoderm.

Examples of cases, in which the ability of the cell obtained in the second step to differentiate into a mesoderm is more improved than the ability of the cell obtained in the first step to differentiate into a mesoderm, include cases in which a relative expression level of T in a cell obtained by differentiating the cell obtained in the second step into an endoderm is preferably 2 or more, and more preferably 3 or more, 5 or more, 8 or more, 9 or more, or 10 or more with respect to an expression level of T in a cell obtained by differentiating the cell obtained in the first step into a mesoderm.

Examples of cases, in which the ability of the cell obtained in the second step to differentiate into a mesoderm is more improved than the ability of the cell obtained in the first step to differentiate into a mesoderm, include cases in which a relative expression level of PDGFRA in a cell obtained by differentiating the cell obtained in the second step into an endoderm is preferably 1.1 or more, and more preferably 1.2 or more, 1.3 or more, or 1.4 or more with respect to an expression level of PDGFRA in a cell obtained by differentiating the cell obtained in the first step into a mesoderm.

Examples of cases, in which the ability of the cell obtained in the second step to differentiate into an ectoderm is more improved than the ability of the cell obtained in the first step to differentiate into an ectoderm, include cases in which a relative expression level of PAX6 in a cell obtained by differentiating the cell obtained in the second step into an ectoderm is preferably 1.1 or more, and more preferably 1.2 or more, 1.3 or more, 1.4 or more, or 1.5 or more with respect to an expression level of PAX6 in a cell obtained by differentiating the cell obtained in the first step into an ectoderm.

Examples of cases, in which the ability of the cell obtained in the second step to differentiate into an ectoderm is more improved than the ability of the cell obtained in the first step to differentiate into an ectoderm, include cases in which a relative expression level of MAP2 in a cell obtained by differentiating the cell obtained in the second step into an ectoderm is preferably 1.1 or more, and more preferably 1.2 or more, or 1.3 or more with respect to an expression level of MAP2 in a cell obtained by differentiating the cell obtained in the first step into an ectoderm.

### [Regarding third step]

The method for producing a cell according to the present invention may further include a third step of differentiating the cell obtained in the second step. However, the method for producing a cell according to the present invention may not include the above-mentioned third step.

In the present invention, the type of cell to be obtained by differentiation-inducing of the cell obtained in the second step is not particularly limited. The cell can be differentiation-induced into an endodermal cell, a mesodermal cell, or an ectodermal cell as necessary.

A method of differentiation-inducing of the cell obtained in the second step is not particularly limited. For example, the cell can be differentiation-induced into each of an endoderm, a mesoderm, and an ectoderm using a commercially available STEMdiff (registered trademark) Trilineage Differentiation Kit (STEMCELL Technologies Inc.).

In addition, differentiation-induction into hemocytes can be performed by culturing the cell under conditions described in Example 2 to be described later. Specifically, the differentiation-induction into hemocytes can be performed as follows: cells are cultured in a medium containing BMP4 and Y27634 (a ROCK inhibitor) on day 1; bFGF and BMP4 are added on day 2; spheroid-like colonies are checked to be formed in the cells on day 3; the cells are cultured in a medium containing SB431542 (a TGF-β receptor inhibitor), CHIR99021 (a GSK3 inhibitor), bFGF, and BMP4 (on day 3 and day 4); the cells are cultured in a medium containing VEGF and bFGF on day 5 and day 6; and the cells are cultured in a medium containing VEGF, bFGF, IL-6, IGF-1, IL-11, and SCF on day 7 to day 10. The differentiation-induction into hemocytes can be checked by analyzing expression of CD34 and KDR, which are markers of hemocytes, by a flow cytometer.

Differentiation-induction into cardiomyocytes can be performed by, for example, culturing cells under conditions described in Example 5 to be described later. Specifically, cells can be differentiation-induced into cardiomyocytes using a PSC Cardiomyocyte Differentiation Kit (Thermo Fisher Scientific) according to a procedure manual. The differentiation-induction into cardiomyocytes can be checked by measuring expression of Cardiac Troponin T (cTnT), which is a marker of cardiomyocytes, by flow cytometry. According to procedures of Example 5 to be described later, the inside of cells after 14 days from differentiation-induction are stained with a Alexa Fluor 647 Mouse Anti-Cardiac Troponin T antibody (BD Pharmingen), and a positive percentage of cTnT is analyzed by a flow cytometer Attune NxT (Thermo Fisher Scientific). A positive percentage of cTnT analyzed by the above method is preferably 5% or more, more preferably 10% or more, even more preferably 20% or more, still more preferably 30% or more, particularly preferably 40% or more, and most preferably 45% or more. By performing the treatment of the present invention (that is, the second step of treating the cell while maintaining an undifferentiated state of the cell to obtain a cell having a relatively high ability to differentiate into a specific differentiated cell), a positive percentage of cTnT is preferably increased by 1.1 to 100 times, more preferably increased by 1.2 to 100 times, and most preferably increased by 2 to 50 times as compared to a case in which the above treatment is not performed.

Differentiation-induction into neural stem cells can be performed by, for example, culturing cells under conditions described in Example 6 to be described later. Specifically, cells can be differentiation-induced into neural stem cells using a PSC Neural Induction Medium (Thermo Fisher Scientific) according to a procedure manual. The differentiation-induction into neural stem cells can be checked by, for example, immunostaining of a SOX1 protein, which is a marker of neural stem cells. In a case where brightness is quantified by immunostaining of the SOX1 protein, by performing the treatment of the present invention (that is, the second step of treating the cell while maintaining an undifferentiated state of the cell to obtain a cell having a relatively high ability to differentiate into a specific differentiated cell), brightness is preferably increased by 1.1 to 10 times, and more preferably increased by 1.5 to 5 times as compared to a case in which the above treatment is not performed.

The cell obtained in the second step can be differentiated into an endodermal cell by culturing the cell under differentiation conditions for endodermal cells. Examples of endodermal cells include digestive system cells (hepatocytes, bile duct cells, pancreatic endocrine cells, acinar cells, duct cells, absorbent cells, goblet cells, paneth cells, intestinal endocrine cells, and the like), and cells of tissues such as lung and thyroid, but examples are not particularly limited thereto.

The cell obtained in the second step can be differentiated into a mesodermal cell by culturing the cell under differentiation conditions for mesodermal cells which are conditions other than the above-described conditions. Examples of mesodermal cells include blood cells and lymphoid cells (hematopoietic stem cells, red blood cells, platelets, macrophages, granulocytes, helper T cells, killer T cells, B lymphocytes, and the like), vascular cells (such as vascular endothelial cells), cardiomyocytes (such as atrial muscle cells and ventricular muscle cells), osteoblasts, bone cells, chondrocytes, tendon cells, adipocytes, skeletal muscle cells, smooth muscle cells, and the like, but examples are not particularly limited thereto.

The cell obtained in the second step can be differentiated into an ectodermal cell by culturing the cell under differentiation conditions for ectodermal cells. Examples of ectodermal cells include nervous cells, sensory organ cells (such as lens, retina, and inner ear), skin epidermal cells, hair follicles, and the like, but examples are not particularly limited thereto.

In the present invention, a cell that has been differentiation-induced using the cell obtained in the second step can be used for screening of candidate compounds for pharmaceuticals for treating various diseases. For example, evaluation can be performed by adding a candidate compound for pharmaceuticals alone or in combination with other drugs to differentiation-induced cells, and detecting morphology or functional changes of cells, increase or decrease of various factors, gene expression profiling, and the like. The cells used herein are preferably cell having the same phenotype as a disease to be treated, and are more preferably cells obtained by differentiation-induction of cells produced by the method of the present invention using somatic cells derived from a patient suffering from the disease.

In the present invention, a tissue can be produced from a cell that has been differentiation-induced using the cell obtained in the second step, and it can used in the field of regenerative medicine. A method of transplanting the prepared tissue into a patient is obvious to those skilled in the art.

The present invention will be described more specifically with reference to the following examples, but the present invention is not limited to the examples.

### Examples

### Example 1

The following experiment was performed to enhance and improve an ability of human iPS cells to differentiate into three germ layers.

### [Method]

### <Cell>

As human iPS cell lines, the line 253G1 and the line 201B7 were purchased from iPS PORTAL, Inc. (Takahashi K, et al. Cell. 2007, Nakagawa M, et al. Nat Biotechnol. 2008). The line A was provided by Cellular Dynamics International (CDI).

### <Cell culture and compound treatment>

Human iPS cells were maintained and cultured in a StemFlex (registered trademark) (Thermo Fisher Scientific) medium under conditions of 37°C, 5% CO₂, and 10% O₂ on a 6-well plate coated with Matrigel (Matrigel) (registered trademark). The cells thus obtained were primed pluripotent stem cells and were cells having a relatively low ability to differentiate into specific differentiated cells.

Day 0: The human iPS cells were detached during the culture by a treatment with TrypLE™ Select (Invitrogen) at 37°C for 5 minutes to form single cells, in order to examine an effect of improving a differentiation ability. The human iPS cells were seeded at 1 x 10⁵ cells/well in a medium in which Y-27684 (10 µmol/L, Wako) was added to mTeSR (registered trademark) 1 (STEMCELL Technologies Inc.) or StemFlex (registered trademark) on a well in which mouse embryonic fibroblasts (MEF, Lonza) had been seeded at 0.5 × 10⁶ cells/well (6-well plate) or a well coated with Matrigel. Thereafter, the cells were cultured under conditions of 37°C, 5% CO₂, and 5% O₂ until day 9.

Day 1: The medium was replaced with a medium 1 in Table 1.

Day 2 to day 3: Half of the medium was replaced with a fresh medium 1.

Day 4 to day 8: The medium was replaced with a medium 2 in Table 1, and the medium was replaced with the medium 2 every day until day 8.

Day 9: The cells were treated with TrypLE™ Select at 37°C for 5 minutes to detach the cells, and subcultured in a medium in which the medium 2 was supplemented with Y-27684 (10 µmol/L, Wako) on a MEF-seeded plate or a plate coated with Matrigel (registered trademark). Thereafter, the cells were maintained and cultured in the medium 2.

**[Table 1]**

| | | | |
|---|---|---|---|
| N2B27 | DMEM/F12 | Thermo Fisher Scientific | 497 mL |
| | Neurobasal (registered trademark) | | 487 mL |
| | B27 (registered trademark) | | 10 mL |
| | N2 | | 5 mL |
| | 1-Thioglycerol | Sigma Aldrich | 10 mL |
| | GlutaMAX (registered trademark) | Thermo Fisher Scientific | 5 mL |
| | Total | | 1014 mL |
| | | | |
| Medium 1 | N2B27 | | |
| | PD0325901 | Wako | 1 µmol/L |
| | hLIF | Peprotech | 1 ng/mL |
| | Valproic acid | Sigma Aldrich | 1 mmol/L |
| | | | |
| Medium 2 | N2B27 | | |
| | PD0325901 | Wako | 1 µmol/L |
| | Gö6983 | TOCRIS | 2 µmol/L |
| | XAV939 | Sigma Aldrich | 2 µmol/L |
| | hLIF | Peprotech | 1 ng/mL |

### <Differentiation into three germ layers>

The cells treated as described above were cultured on a Matrigel-coated plate using StemFlex (registered trademark) for 4 to 7 days until the cells proliferated to reach the required number of cells in order to examine an ability to differentiate into three germ layers. For differentiation into three germ layers, human iPS cells that had not been treated or were subjected to the above treatment were differentiated using a STEMdiff (registered trademark) Trilineage Differentiation Kit (STEMCELL Technologies Inc.) according to a procedure manual.

Day 1: The cells were treated using TrypLE™ Select (Thermo Fisher Scientific) at 37°C for 5 minutes to detach the cells. 0.5 mL/well of a medium of Table 2 was added to a Matrigel-coated 24-well plate, and iPS cells were seeded and cultured under the conditions of 37°C, 5% CO₂, and 10% O₂.

Day 2: The medium was replaced with a new medium of a STEMdiff (registered trademark) Trilineage Differentiation Kit for each of differentiation. Thereafter, the same operation was repeated every day until day 5 for an endoderm and a mesoderm and until day 7 for an ectoderm.

**[Table 2]**

| | The number of cells/well | Medium |
|---|---|---|
| Ectoderm | 4 × 10⁵ | STEMdiff (registered trademark) Trilineage Ectoderm medium + 10 µmol/L of Y-27632 |
| Mesoderm | 1 × 10⁵ | mTeSR (registered trademark) 1 + 10 µmol/L of Y-27632 |
| Endoderm | 4 × 10⁵ | mTeSR (registered trademark) 1 + 10 µmol/L of Y-27632 |

<Evaluation of undifferentiation state and ability to differentiate into three germ layers by quantitative RT-PCR>

Total RNA was extracted from iPS cells and cells differentiated into three germ layer lineages using an RNeasy (registered trademark) Mini Kit (QIAGEN), and a reverse transcription reaction was performed using a High-Capacity RNA-to-cDNA™ Kit (Applied Biosystems) to synthesize cDNA. TaqMan (registered trademark) gene expression assay (Applied Biosystems) and TaqMan (registered trademark) Fast Advanced Master Mix (Applied Biosystems) of genes as indicators for an undifferentiation state or genes specific to each of germ layers shown in Table 3 were added to the synthesized cDNA, and a PCR reaction was performed using Viia7™ (Applied Biosystems). The letters shown in Probe primer set in Table 3 are code names of Probe primer sets for performing PCR of genes in Taqman (registered trademark) Gene expression assay of Thermo Fisher.

**[Table 3]**

| | Gene symbol | Probe primer set |
|---|---|---|
| Undifferentiated state | *NANOG* | Hs02387400_gl |
| | *POU5F1* | Hs04260367_gH |
| | *LIN28* | Hs00702808_s1 |
| | *SOX2* | Hs00415716_m1 |
| | *DNMT3B* | Hs00171876_m1 |
| | *KLF17* | Hs00702999_m1 |
| | *STELLA* | Hs01931905_gl |
| Ectoderm | *PAX6* | Hs01088114_m1 |
| | *MAP2* | Hs00258900_m1 |
| Mesoderm | *T* | Hs00610080_m1 |
| | *PDGFRA* | Hs00998018_m1 |
| Endoderm | *SOX17* | Hs00751752_s1 |
| | *FOXA2* | Hs00232764_m1 |

### [Results]

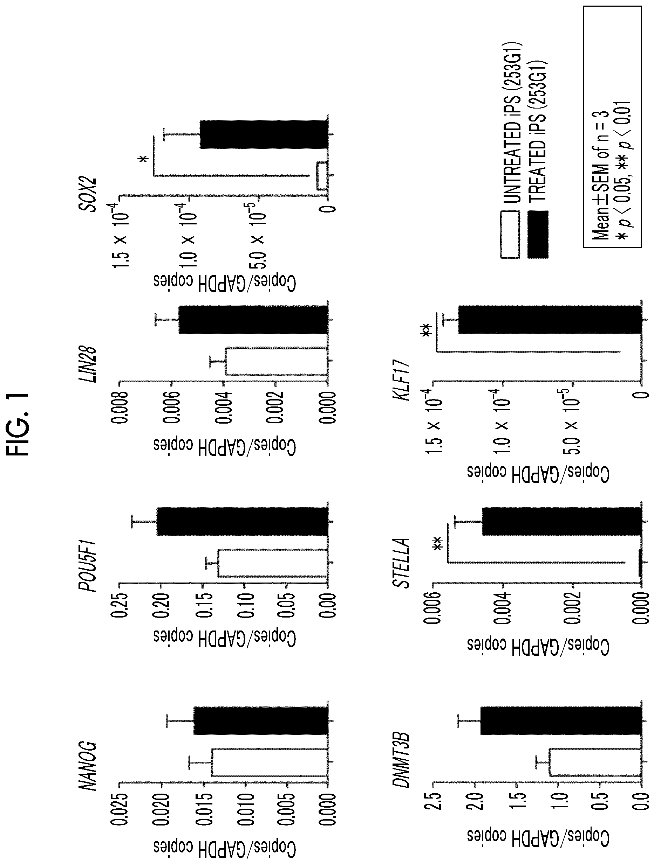

Fig. 1 shows results of quantitative RT-PCR for measuring expression of genes defining an undifferentiation state in untreated and treated human iPS cell lines (the line 253G1).

As shown in Fig. 1, expression of NANOG, POU5F1, LIN28, and DNMT3B was not significantly changed between the untreated cells and the treated cells. On the other hand, as can be seen from Fig. 1, expression of STELLA and KLF17, which are specifically expressed in SOX2 and naive ES/iPS cells, was significantly increased in the treated iPS cells.

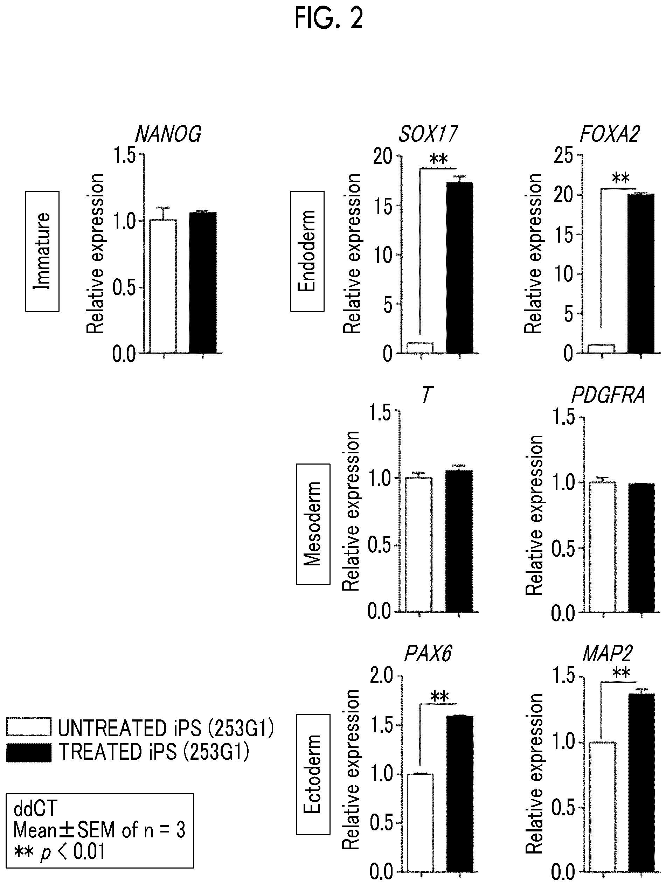

Fig. 2 shows results of quantitative RT-PCR for measuring expression of genes specific to each of germ layers in cells (the line 253G1) differentiated into three germ layer lineages.

As shown in Fig. 2, expression of the endoderm-specific genes (SOX17 and FOXA2) and the ectoderm-specific genes (PAX6 and MAP2) was significantly increased in the treated cells as compared to the untreated cells.

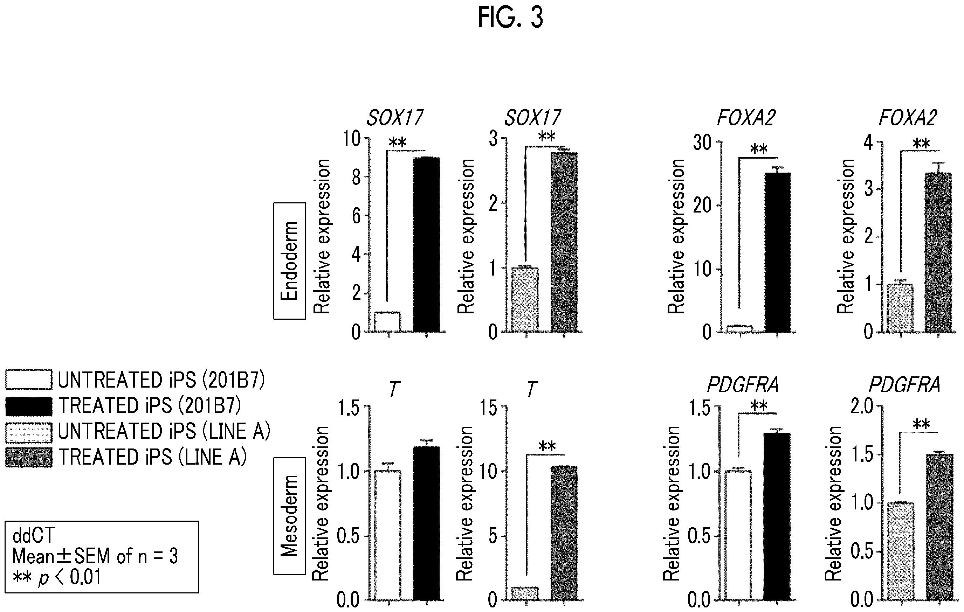

Fig. 3 shows results of quantitative RT-PCR for measuring expression of genes specific to each of germ layers in another iPS cell lines (the line 201B7 and the line A) differentiated into three germ layer lineages.

As shown in Fig. 3, in the other iPS cell lines (201B7 and A), expression of the endoderm-specific genes (SOX17 and FOXA2) and the mesoderm-specific genes (T and PDGFRA) was significantly increased in the treated cells as compared to the untreated cells.

Based on the above results, it was found that the above treatment of iPS cells improved an ability to differentiate into three germ layers. In this case, it became clear that in the evaluated and examined iPS cell lines, a differentiation ability of some cells deteriorated in a culture method of the related art, but by subjecting the cells to the above treatment, the deteriorated differentiation ability was ameliorated.

### Example 2

An influence of the treatment performed in Example 1 on an ability to differentiate into hemocytes as one type of mesodermal lineage was verified.

### [Method]

### <Cell>

An iPS cell B line used was provided by CDI.

### <Cell culture and compound treatment>

The culture and treatment were performed in the same manner as in Example 1.

### <Differentiation into hemocytes>

Day 1: The cells were treated using TrypLE™ Select at 37°C for 5 minutes to detach the cells. The cells were suspended in a medium of Day 1 in Table 4, and 2 × 10⁶ cells of iPS cells were seeded on one well of a EZSPHERE (registered trademark) (AGC) 6-well plate for spheroid formation. Thereafter, the cells were cultured under conditions of 37°C, 5% CO₂, and 5% O₂.

Day 2: bFGF (5 ng/mL) and BMP4 (10 ng/mL) were added.

Day 3: Spheroidal colonies were checked to be formed in the cells, and the medium was replaced with a medium of Day 3.

Day 5: The medium was replaced with a medium of Day 5.

Day 7: The medium replaced with a medium of Day 7.

Day 8 to day 10: The medium was replaced with a medium of Day 7 every day.

**[Table 4]**

| Supplemented StemPro (registered trademark)-34 | | |
|---|---|---|
| StemPro (registered trademark)-34 | Thermo Fisher Scientific | 490.5 mL |
| L-Glutamine | Thermo Fisher Scientific | 5 mL |
| (+)-Sodium L-ascorbate | Sigma Aldrich | 0.5 mL |
| holo-Transferrin Human | Sigma Aldrich | 7.5 mL |
| 1-Thioglycerol (50mmol/L) | Sigma Aldrich | 4 mL |
| Total | | 507.5 mL |

| Dayl | | |
|---|---|---|
| Supplemented StemPro (registered trademark)-34 | | |
| BMP4 | Peprotech | 10 ng/mL |
| Y-27634 | Wako | 10 µmol/L |

| Day3 | | |
|---|---|---|
| Supplemented StemPro (registered trademark)-34 | | |
| SB431542 | Wako | 6 µmol/L |
| CHIR99021 | TOCRIS | 3 µmol/L |
| bFGF | Peprotech | 5 ng/mL |
| BMP4 | Peprotech | 10 ng/mL |

| Day5 | | |
|---|---|---|
| Supplemented StemPro (registered trademark)-34 | | |
| VEGF | Peprotech | 15 ng/mL |
| bFGF | Peprotech | 5 ng/mL |

| Day7 | | |
|---|---|---|
| Supplemented StemPro (registered trademark)-34 | | |
| VEGF | Peprotech | 15 ng/mL |
| bFGF | Peprotech | 10 ng/mL |
| IL-6 | Peprotech | 10 ng/mL |
| IFG-1 | Peprotech | 25 ng/mL |
| IL-11 | Peprotech | 5 ng/mL |
| SCF | Peprotech | 50 ng/mL |

### <Detection or hemocytes by flow cytometry>

The spheroid-shaped cells were recovered, and the cells were converted into single cells by treatment with TrypLE™ Select at 37°C for 5 minutes. The cells were stained with Phycoerythrin (PE)-Cy7 anti-human CD34 (BioLegend) (where Cy is registered trademark) and PE anti-human CD309 (KDR) (BioLegend), were analyzed by a flow cytometer (Attune (registered trademark) NxT; Thermo Fisher Scientific, and FACSAria (registered trademark) III; BD Bioscience).

### [Results]

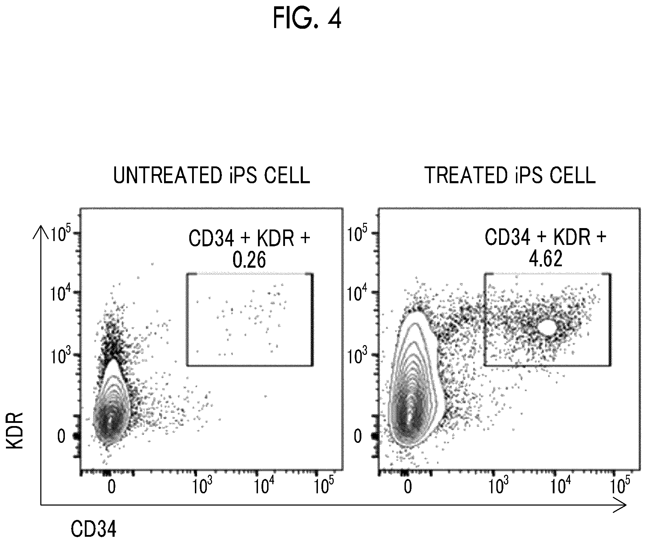

Fig. 4 shows results obtained by respectively differentiating untreated and treated iPS cell B lines into hemocytes, and analyzing a proportion of cells expressing CD34 and KDR, which are markers of hemocytes, by a flow cytometer. As shown in Fig. 4, a proportion of CD34- and KDR-positive cells increased about 17.7-fold in a case where cells differentiated from the treated iPS cells as compared to the untreated cells.

### Example 3

In order to verify necessity of Valproic acid (VPA) and bFGF in the treatment for improving an ability to differentiate into three germ layers, the following experiment was performed.

### [Method]

### <Cell>

A human iPS cell 253G1 line used was purchased from iPS PORTAL, Inc.

### <Cell culture and compound treatment>

The human iPS cells were maintained in a StemFlex (registered trademark) medium under conditions of 37°C, 5% CO₂, and 10% O₂ on a Matrigel-coated 6-well plate.

Day 0: The human iPS cells were detached during the culture by a treatment with TrypLE™ Select at 37°C for 5 minutes to form single cells, in order to examine an effect of improving a differentiation ability. The human iPS cells were seeded at 1 × 10⁵ cells/well in a medium in which Y-27684 (10 µmol/L, Wako) was added to StemFlex (registered trademark) in one well of a Matrigel-coated 6-well plate. Thereafter, the cells were cultured under conditions of 37°C, 5% CO₂, and 5% O₂ until day 9.

Day 1: The medium was replaced with a medium 1 in Table 5.

Day 2 to day 3: Half of the medium was replaced with a fresh medium 1.

Day 4 to day 8: The medium was replaced with a medium 2 in Table 5, and the medium was replaced with the medium 2 every day until day 8.

Day 9: For cells under all conditions, the medium was replaced with StemFlex (registered trademark), and the cells were cultured for about 4 days.

**[Table 5]**

| | Condition (1) | |
|---|---|---|
| Medium 1 | N2B27 | |
| | Valproic acid | 1 mmol/L |
| | PD0325901 | 1 µmol/L |
| | Gö6983 | 2 µmol/L |
| | XAV939 | 2 µmol/L |
| | hLIF | 10 ng/mL |
| | | |
| Medium 2 | N2B27 | |
| | PD0325901 | 1 µmol/L |
| | Gö6983 | 2 µmol/L |
| | XAV939 | 2 µmol/L |
| | hLIF | 10 ng/mL |

| Condition (2) | | |
|---|---|---|
| N2B27 | | |
| Valproic acid | | 1 mmol/L |
| PD0325901 | | 1 µmol/L |
| Gö6983 | | 2 µmol/L |
| XAV939 | | 2 µmol/L |
| bFGF | | 10 ng/mL |
| | | |

| N2B27 | | |
|---|---|---|
| PD0325901 | | 1 µmol/L |
| Gö6983 | | 2 µmol/L |
| XAV939 | | 2 µmol/L |
| bFGF | | 10 ng/mL |

| Condition (3) | | |
|---|---|---|
| N2B27 | | |
| | | |
| PD0325901 | | 1 µmol/L |
| Gö6983 | | 2 µmol/L |
| XAV939 | | 2 µmol/L |
| hLIF | | 10 ng/mL |
| | | |

| N2B27 | | |
|---|---|---|
| PD0325901 | | 1 µmol/L |
| Gö6983 | | 2 µmol/L |
| XAV939 | | 2 µmol/L |
| hLIF | | 10 ng/mL |

### <Differentiation into three germ layers>

The culture and treatment were performed in the same manner as in Example 1.

### <Evaluation of ability to differentiate into three germ layers by quantitative RT-PCR>

The culture and treatment were performed in the same manner as in Example 1.

### [Results]

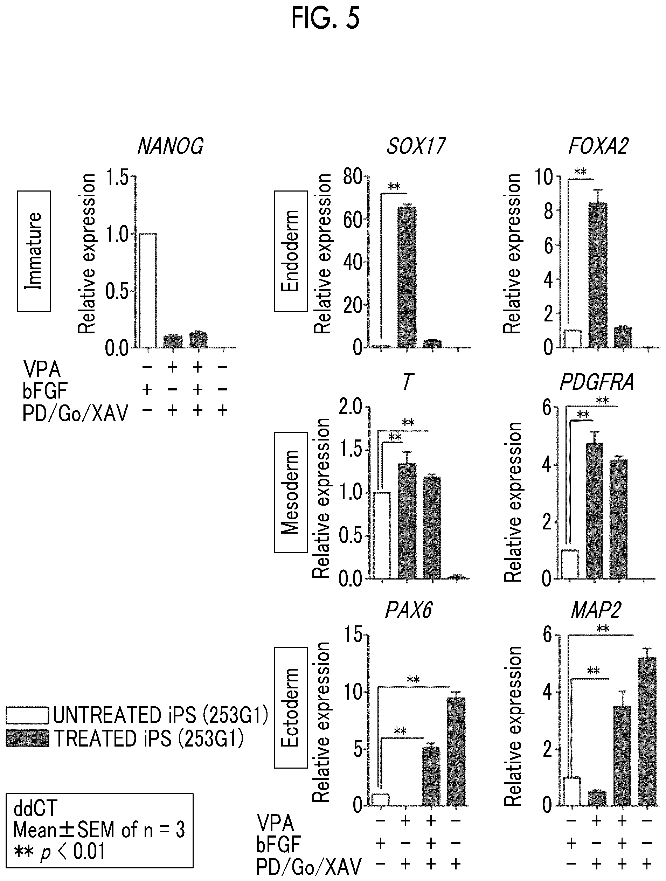

Fig. 5 shows results of quantitative RT-PCR for evaluating an ability to differentiate into three germ layers.

Regarding differentiation of the line 253G1 into an endoderm, expression of endoderm-specific genes (SOX17 and FOXA2) was significantly increased by the treatment with the condition (1): Valproic acid (VPA) + inhibitors (PD0325901, Go6983, and XAV939). Since this increase in the gene expression was not recognized in the case in which bFGF was added to the condition (1) (condition (2)) and the case in which VPA was removed (condition (3)), and therefore it was found that addition of VPA and removal of bFGF were necessary to improve differentiation into an endoderm.

Next, regarding differentiation into a mesoderm, by the treatment with the condition (1): VPA + inhibitors and the condition (2): VPA + inhibitors + bFGF, expression of mesoderm-specific genes (T and PDGFRA) was significantly increased. On the other hand, the gene expression was decreased in the case in which VPA was removed as the condition (3), and therefore it was found that addition of VPA was necessary to improve differentiation into a mesoderm.

Regarding differentiation into an ectoderm, an increase in expression of ectoderm-specific genes (PAX6 and MAP2) was not recognized in the case of the treatment with the condition (1): VPA + inhibitors, whereas a significant increase in expression thereof was recognized in the case of the condition (2): VPA + inhibitors + bFGF and the condition (3): only inhibitors. Accordingly, it was found that addition of bFGF was necessary to differentiate VPA-treated iPS cells into an ectoderm.

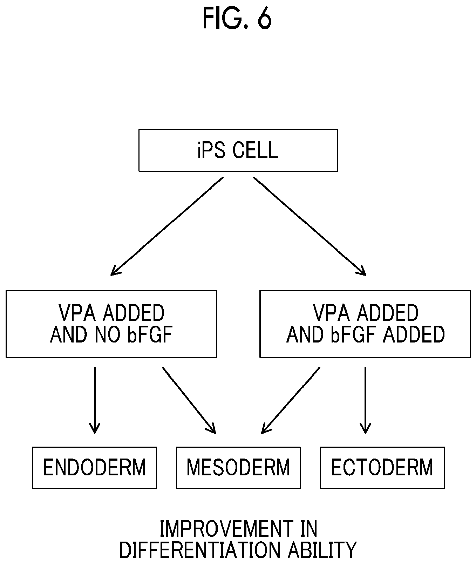

Fig. 6 shows a summary of the results of Fig. 5.

Based on the above results, it was found that an ability to differentiate into an endoderm was improved by subjecting human iPS cells to the VPA treatment and removing bFGF, an ability to differentiate into an ectoderm was improved by the treatment with VPA and bFGF, and an ability to differentiate into a mesoderm was improved by any of the treatments.

### Example 4

An influence of a difference in concentrations of a MEK inhibitor PD0325901 in human iPS cells on the treatment effect was verified.

### [Method]

### <Cell>

As human iPS cell lines, the line 253G1 and the line 201B7 were provided by Center for iPS Cell Research and Application, Kyoto University (Takahashi K, et al. Cell. 2007, Nakagawa M, et al. Nat Biotechnol. 2008). The line C and the line D were provided by Cellular Dynamics International (CDI).

### <Cell culture and compound treatment>

The culture and the treatment were performed in the same manner as in Example 1 except that the treatment was performed under conditions in which a concentration of PD0325901 was 0.3 to 1.0 µmol/L, and subculture was performed for about 2 weeks after day 9.

### <Method of evaluating treatment efficiency by flow cytometry>

The completion of the treatment in Example 1 was evaluated by a positive percentage of a cell surface marker CD75. The cultured human iPS cells were detached with TrypLE™ Select, and stained with Alexa Fluor 647 Mouse Anti-Human CD75 antibody. A positive percentage of CD75 was analyzed by a flow cytometer Attune NxT.

### <Evaluation of number of copies of specific region of chromosome>

The number of copies of a region in which DNA mutations easily occur in human pluripotent stem cells was evaluated by a real-time PCR method to confirm an influence of a difference in concentrations of PD0325901 added to iPS cells on an incidence of DNA mutations. Genomic DNA was extracted from iPS cells using a Pure Link Genomic DNA Mini Kit (Thermo Fisher Scientific). The extracted DNA was subjected to real-time PCR with ViiA7 (Thermo Fisher Scientific) using a hPSC Genetic Analysis Kit (STEMCELL Technologies Inc.) to calculate the number of copies of a chromosomal region.

### [Results]

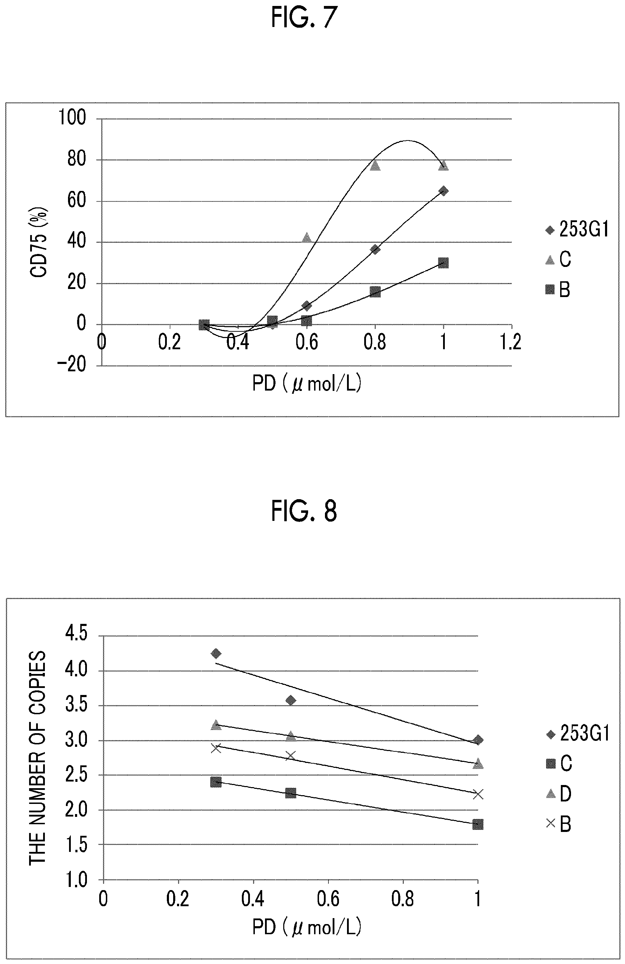

Fig. 7 shows results of evaluating treatment efficiency by flow cytometry. As a result of treating of the three lines of human iPS cells with PD0325901 at concentrations of 0.3 to 1.0 µmol/L, there was an increase in positive percentage of CD75, which an indicator showing that treating of the all three lines was completed in a PD0325901-concentration-dependent manner (Fig. 7). Since CD75 was not positive in any of the cell lines within the range of 0.3 to 0.5 µmol/L, it was found that a concentration of PD0325901 was desirably 0.6 to 1.0 µmol/L.

Fig. 8 shows results of evaluating the number of copies of a specific region of chromosome. As a result of calculating the number of copies of a long arm region of chromosome 20 by real-time PCR, the number of copies decreased in a PD0325901-concentration-dependent manner and approached the normal number of copies, which is 1.5 to 2.5 (Fig. 8). Based on the above results, it was found that it is desirable to add PD0325901 at a concentration of 0.8 to 1.2 µmol/L to suppress DNA mutations to a minimum level.

### Example 5

An influence of the treatment performed in Example 1 on an ability of human iPS cells to differentiate into cardiomyocytes was verified.

### [Method]

### <Cell>

As a human iPS cell line, the line 253G1 was provided by Center for iPS Cell Research and Application, Kyoto University (Takahashi K, et al. Cell. 2007, Nakagawa M, et al. Nat Biotechnol. 2008). The lines D, E, G, H, and I were provided by Cellular Dynamics International (CDI).

### <Cell culture and compound treatment>

The human iPS cells were treated in the same manner as in Example 1, and subcultured in the medium 2 for about 2 weeks. Thereafter, the treated cells were subcultured on a Matrigel-coated plate, and subcultured in a StemFlex or mTeSR1 medium for about 2 weeks.

### <Differentiation into cardiomyocytes>

Human iPS cells that had not been treated or were subjected to the above treatment were differentiated using a PSC Cardiomyocyte Differentiation Kit (Thermo Fisher Scientific) according to a procedure manual.

### <Evaluation method>

For the efficiency of induction into cardiomyocytes, expression of Cardiac Troponin T (cTnT), which is a marker of cardiomyocytes, was evaluated by flow cytometry. Cells after 14 days from differentiation-induction were detached with TrypLE™ Select, and dead cells were stained with Ghost Dye Violet 510 (TONBO Bioscience). After washing, the cells were fixed and permeated with a BD Cytofix Fixation buffer and a Perm/Wash buffer (BD Bioscience). The inside of the cells were stained with a Alexa Fluor 647 Mouse Anti-Cardiac Troponin T antibody (BD Pharmingen), and a positive percentage of cTnT was analyzed by a flow cytometer Attune NxT (Thermo Fisher Scientific).

### [Results]

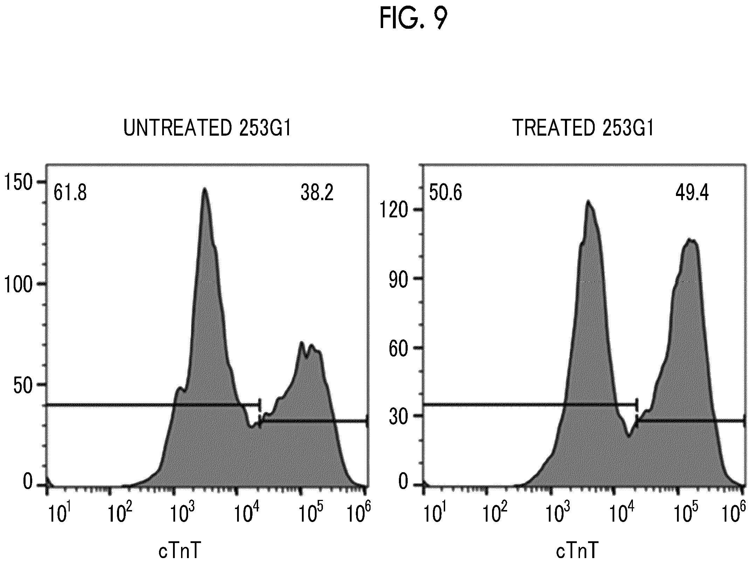

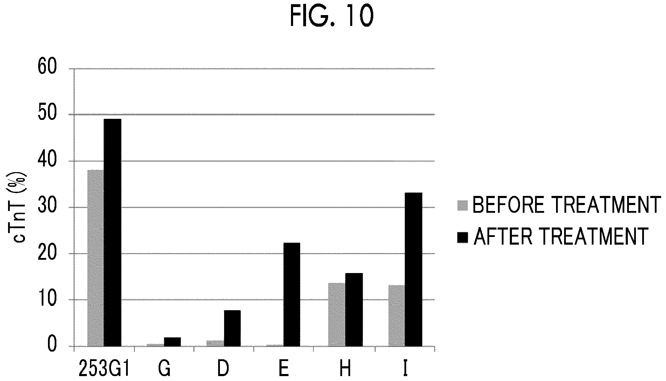

Fig. 9 shows results obtained by respectively inducing untreated and treated iPS cells into cardiomyocytes, and comparing cTnT-positive percentages in living cells by flow cytometry. A positive percentage in the untreated cells was 38.2%, and a positive percentage in the treated cells was 49.4%, which was a 1.29-fold increase (Fig. 9). In addition, Fig. 10 shows results of measuring cTnT-positive percentages in a case where 5 iPS cell lines were also induced into cardiomyocytes under the same conditions. A cTnT-positive percentage increased in 5 lines (Fig. 10).

### Example 6

An effect of the method of the present invention for improving differentiation into neural stem cells was verified.

### [Method]

### <Cell>

Five lines (the line J, the line K, the line L, the line M, and the line N) provided from Cellular Dynamics International (CDI) were treated by the method of Example 1 to obtain cells.

### <Induction into neural stem cells>

The cells subjected to the above treatment were cultured on a plate coated with Geltrex (Thermo Fisher Scientific) for 1 to 2 days using mTeSR (registered trademark) until the cells proliferated to reach the required number of cells in order to examine an ability to differentiate into neural stem cells. For differentiation into neural stem cells, human iPS cells that had not been treated or were subjected to the above treatment were differentiated using a PSC Neural Induction Medium (Thermo Fisher Scientific) according to a procedure manual.

### <Evaluation>

On day 7 from the induction, the cells were treated using TrypLE™ Select or Accutase (Thermo Fisher Scientific) at 37°C for 5 minutes to detach the cells. 3 × 10⁵ cells/well of the cells were seeded on a 8-well chamber plate coated with Geltrex, and the cells were cultured under conditions of 37°C, 5% CO₂, and 10% O₂. After 24 hours, a SOX1 protein, which is a marker of neural stem cells, was immunostained using a Human Neural Stem Cell Immunocytochemistry Kit (Thermo Fisher Scientific). The observation was performed using a fluorescence microscope (Keyence).

### <Image quantification>

For random 20 cells, brightness from images of the fluorescence microscope was quantified by ImageJ (NIH). Average values thereof are shown in a graph.

### [Results]

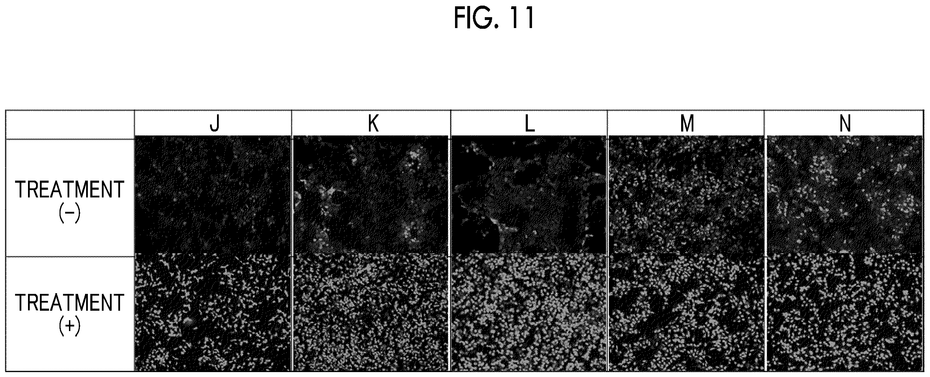

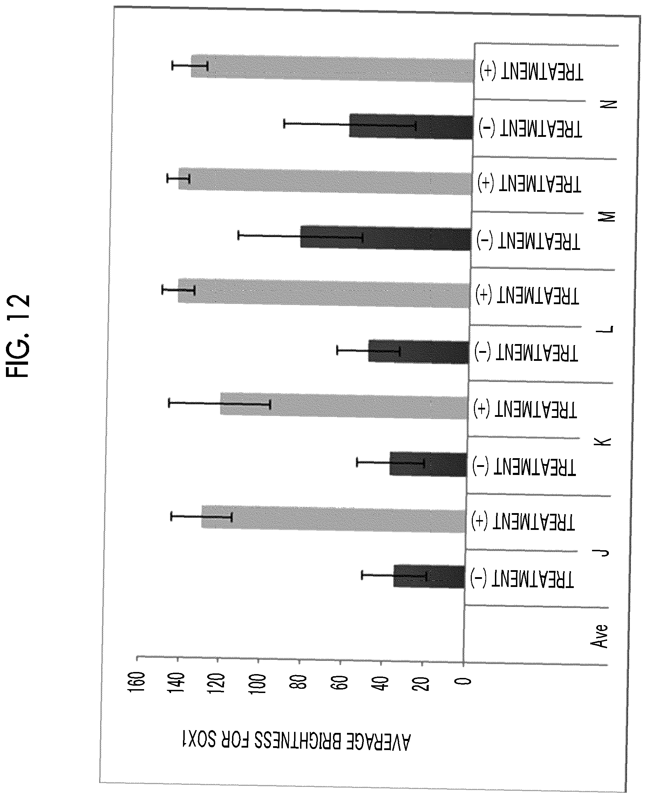

Fig. 11 shows results of observation with the fluorescence microscope, and Fig. 12 shows results of quantifying brightness of the images. Expression of SOX1 was increased and homogenized before and after the treatment in the five cells of the five cells. It was found that there was significant improvement in the five lines. After the treatment, the brightness was increased by a factor of 3 on average (Figs. 11 and 12).

## Claims

1. A method for producing a cell, comprising:
a first step of obtaining an undifferentiated cell which is obtained by introducing a reprogramming factor into a somatic cell and has a relatively low ability to differentiate into a specific differentiated cell; and
a second step of treating the cell while maintaining an undifferentiated state of the cell to obtain a cell having a relatively high ability to differentiate into the specific differentiated cell.

2. The method according to claim 1, wherein the ability of the cell obtained in the second step to differentiate into any one or more of an endoderm, a mesoderm, or an ectoderm is more improved than the ability of the cell obtained in the first step to differentiate into any one or more of an endoderm, a mesoderm, or an ectoderm.

3. The method according to claim 1,
wherein the ability of the cell obtained in the second step to differentiate into each of an endoderm and an ectoderm is more improved than the ability of the cell obtained in the first step to differentiate into each of an endoderm and an ectoderm, or
the ability of the cell obtained in the second step to differentiate into each of an endoderm and a mesoderm is more improved than the ability of the cell obtained in the first step to differentiate into each of an endoderm and a mesoderm.

4. The method according to claim 2 or 3, wherein the cell obtained in the second step is a cell capable of differentiating into all of an endoderm, a mesoderm, and an ectoderm.

5. The method according to claim 1, wherein the specific differentiated cell is a mesodermal cell or an ectodermal cell.

6. The method according to claim 1, wherein the specific differentiated cell is a hemocyte, a cardiomyocyte, or a neurocyte.

7. The method according to any one of claims 1 to 6, wherein an expression level of STELLA in the cell obtained in the second step is higher than an expression level of STELLA in the cell obtained in the first step.

8. The method according to any one of claims 1 to 7, wherein an expression level of KLF17 in the cell obtained in the second step is higher than an expression level of KLF17 in the cell obtained in the first step.

9. The method according to any one of claims 1 to 8, wherein the second step is a step of treating the cell with a histone deacetylase inhibitor while maintaining the undifferentiated state of the cell.

10. The method according to claim 9, wherein the second step is a step of treating the cell with the histone deacetylase inhibitor and a basic fibroblast growth factor while maintaining the undifferentiated state of the cell.

11. The method according to claim 9 or 10, wherein the second step includes a step of culturing the cell in a medium containing the histone deacetylase inhibitor, a MAPK/ERK kinase inhibitor, and a leukemia inhibitory factor, and then culturing the cell in a medium not containing the histone deacetylase inhibitor but containing the MAPK/ERK kinase inhibitor, a protein kinase C inhibitor, a Wnt signaling inhibitor, and the leukemia inhibitory factor.

12. The method according to any one of claims 9 to 11, wherein the histone deacetylase inhibitor is valproic acid or a salt thereof.

13. The method according to any one of claims 1 to 12, wherein a medium in the second step does not contain ascorbic acid.

14. The method according to any one of claims 1 to 13, wherein the somatic cell is a human adult somatic cell.

15. The method according to any one of claims 1 to 14, further comprising a third step of differentiating the cell obtained in the second step.

16. A method for producing a pluripotent stem cell having an improved ability to differentiate into three germ layers as compared to a primed pluripotent stem cell, the method comprising a step of treating the primed pluripotent stem cell with a histone deacetylase inhibitor while maintaining an undifferentiated state of the cell.

17. A method for producing a pluripotent stem cell having an improved ability to differentiate into a specific differentiated cell as compared to a primed pluripotent stem cell, the method comprising a step of treating the primed pluripotent stem cell with a histone deacetylase inhibitor while maintaining an undifferentiated state of the cell.

18. The method according to claim 17, wherein the specific differentiated cell is a hemocyte, a cardiomyocyte, or a neurocyte.

19. The method according to any one of claims 16 to 18, wherein the step of treating the primed pluripotent stem cell with the histone deacetylase inhibitor while maintaining the undifferentiated state of the cell includes a step of culturing the cell in a medium containing the histone deacetylase inhibitor, a MAPK/ERK kinase inhibitor, and a leukemia inhibitory factor, and then culturing the cell in a medium not containing the histone deacetylase inhibitor but containing the MAPK/ERK kinase inhibitor, a protein kinase C inhibitor, a Wnt signaling inhibitor, and the leukemia inhibitory factor.

20. The method according to any one of claims 16 to 19, wherein the histone deacetylase inhibitor is valproic acid or a salt thereof.

21. The method according to any one of claims 16 to 20, wherein a medium in the step of treating the primed pluripotent stem cell with the histone deacetylase inhibitor while maintaining the undifferentiated state of the cell does not contain ascorbic acid.
